# FASCICULE DE BREVET EUROPEEN

(11) **EP 1 655 017 B1**
(45) Date de publication et mention de la délivrance du brevet: **30.12.2009**
(21) Numéro de dépôt: 05292307.5
(22) Date de dépôt: 02.11.2005
(51) Int. Cl.: A61K 8/25, A61Q 5/10

(54) **Utilisation de composés de type organosilane en teinture des fibres kératiniques**
Verwendung von Verbindungen vom Typ Organosilan zum Färben keratinischer Fasern
Use of compounds of the type organosilane for dyeing keratinic fibres

(30) Priorité: 03.11.2004 FR 0411714
(43) Date de publication de la demande: 10.05.2006
(73) Titulaire: L'ORÉAL, 75008 Paris (FR)
(72) Inventeur: Kravtchenko, Sylvain, 92600 Asnieres (FR); Lagrange, Alain, 77700 Coupvray (FR)
(74) Mandataire: Casalonga, Axel

(56) Documents cités:
- EP-A- 0 940 404
- FR-A- 2 786 481
- FR-A- 2 787 705
- FR-A- 2 788 433

## Description

La présente demande a pour objet l'utilisation de composés particuliers de type organosilane, et notamment de composés possédant au moins une fonction trialcoxysilane, à titre de colorants directs dans des compositions tinctoriales pour la teinture des fibres kératiniques, et en particulier les cheveux humains. Elle vise également des compositions tinctoriales à base de ces colorants, ainsi que l'utilisation de ces compositions pour la teinture des fibres kératiniques.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. Ces colorants, insolubles dans le milieu de teinture, sont piégés à l'intérieur du cheveu.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques par une coloration directe ou semi-permanente. Le procédé classiquement utilisé en coloration directe consiste à appliquer sur les fibres kératiniques des colorants directs qui sont des molécules colorées et colorantes ayant une affinité pour les fibres, à laisser poser pour permettre aux molécules colorées de pénétrer, par diffusion, à l'intérieur du cheveu, puis à rincer les fibres.

Contrairement aux compositions de teinture par oxydation, les compositions de teinture directes ou semi-permanentes peuvent avantageusement être mises en oeuvre sans la présence d'un agent oxydant. Ces teintures peuvent être effectuées de manière répétée sans dégrader la fibre kératinique.

Il est connu par exemple d'utiliser des colorants directs nitrés benzéniques, anthraquinoniques, nitropyridiniques, azoïques, xanthéniques, acridiniques, aziniques ou triarylméthaniques.

Il en résulte des colorations particulièrement chromatiques qui sont cependant temporaires ou semi-permanentes à cause de la nature des liaisons entre les colorants directs et la fibre kératinique. Ces interactions font que la désorption des colorants de la surface et/ou du coeur de la fibre se fait facilement. Les colorations présentent généralement une faible puissance tinctoriale et une mauvaise tenue aux lavages ou à la transpiration. Ces colorants directs sont en outre généralement sensibles à la lumière car la résistance du chromophore vis-à-vis des attaques photochimiques est faible, ce qui conduit à un affadissement de la coloration des cheveux dans le temps. La sensibilité de ces colorants à la lumière dépend de leur répartition uniforme ou en agrégats dans et/ou sur la fibre kératinique.

Par ailleurs, les colorants directs classiques peuvent ne pas être complètement inoffensifs, c'est pourquoi en cosmétique capillaire, on recherche des molécules colorantes de ce type toujours plus performantes en terme d'innocuité.

Il existe un réel besoin de disposer de compositions de teinture directe améliorées en terme de tenue aux shampooings et de montée du colorant.

De manière surprenante et avantageuse, la Demanderesse vient de découvrir que l'utilisation de composés particuliers de type organosilane dans des compositions pour la teinture des fibres kératiniques, en particulier des fibres kératiniques humaines telles que les cheveux, permettait d'obtenir des compositions tinctoriales qui présentent ces améliorations.

Outre leur avantage en terme d'innocuité, les compositions selon la présente demande permettent l'obtention de teintures résistantes aux agents extérieurs (tels que le soleil, les intempéries), ainsi qu'aux shampooings et à la transpiration, et permettent d'obtenir des reflets intenses et tenaces sur les fibres.

En outre, ces compositions présentent une montée améliorée ainsi qu'un bon profil toxicologique.

La présente invention a ainsi pour objet l'utilisation à titre de colorant direct dans des compositions tinctoriales pour fibres kératiniques, en particulier les fibres kératiniques humaines telles que les cheveux, ou pour la fabrication de telles compositions, d'un composé particulier de type organosilane.

L'invention a également pour objet une composition tinctoriale pour la teinture des fibres kératiniques, en particulier des fibres kératiniques humaines telles que les cheveux comprenant dans un milieu de teinture approprié au moins une base d'oxydation et au moins un composé de type organosilane particulier.

Un autre objet de l'invention porte sur un procédé de teinture des fibres kératiniques, en particulier des fibres kératiniques humaines telles que les cheveux mettant en oeuvre cette composition.

En particulier, le procédé peut consister à appliquer sur les fibres kératiniques une première composition selon l'invention, puis à appliquer sur les fibres kératiniques après un éventuel rinçage une deuxième composition contenant un agent alcalin.

L'invention a également pour objet l'utilisation de la composition de la présente invention sur les fibres kératiniques, en particulier les fibres kératiniques humaines telles que les cheveux pour obtenir des teintures présentant une bonne résistance aux agents extérieurs et aux shampooings.

D'autres caractéristiques, aspects, objets et avantages de l'invention apparaîtront encore plus clairement à la lecture de la description et des exemples qui suivent.

Les composés de type organosilane selon l'invention répondent à la formule (I) suivante : 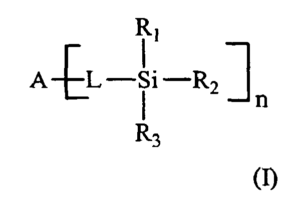 dans laquelle :
le groupe A est un groupe à fonction colorante directe,
le groupe L est un bras de liaison,
R₁, R₂, et R₃, indépendamment les uns des autres, représentent un hydrogène ; un radical mono ou polyhydroxyalkyle ayant de 1 à 10 atomes de carbone ; un radical mono ou polyaminoalkyle ayant de 1 à 10 atomes de carbone ; un radical mono ou polyhalogènealkyle ayant de 1 à 10 atomes de carbone ; un radical mono ou polyalcoxyalkyle, le radical alcoxy étant en C₁-C₁₀ et le radical alkyle en C₁-C₁₀ ; un radical aryle en C₆-C₁₈ ; un radical mono ou polyaminoaryle en C₆-C₁₈ ; un radical mono ou polyhydroxyaryle en C₆-C₁₈ ; un radical mono ou polyalcoxyaryle, le radical alcoxy étant en C₁-C₁₀ et le radical aryle en C₆-C₁₈ ; un radical alkylaryle, le radical alkyle étant en C₁-C₁₀ et le radical aryle en C₆₋C₁₈ ; un radical arylalkyle, le radical alkyle étant en C₁-C₁₀ et le radical aryle en C₆-C₁₈ ; un radical carboxyalkyle ou sulfoalkyle, le radical alkyle étant en C₁-C₁₀,
n est un entier compris entre 1 et 10.

De préférence, les radicaux alkyle sont en C₁-C₆.

De préférence, les composés de type organosilane sont des composés contenant au moins une fonction trialcoxysilane et répondant à la formule (II) suivante : 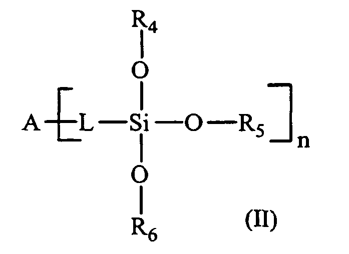 dans laquelle :
le groupe A est un groupe à fonction colorante directe,
le groupe L est un bras de liaison,
R₄, R₅, et R₆, indépendamment les uns des autres, représentent un radical alkyle en C₁-C₁₀, de préférence en C₁-C₆, linéaire ou ramifié, substitué ou non substitué,
n est un entier compris entre 1 et 10.

Le radical alkyle correspondant à R₄, R₅ et R₆ peut notamment être le radical méthyle, éthyle, n-propyle, iso-propyle, ou butyle. De préférence, ce radical alkyle est un radical méthyle ou éthyle.

Le groupe A est un groupe à fonction colorante directe. Par « groupe à fonction colorante directe » au sens de l'invention, on entend un groupe présentant une absorption comprise entre 350 et 800 nm.

Le groupe A peut être choisi parmi les radicaux dérivant des colorants nitro aromatiques, anthraquinoniques, naphtoquinoniques, benzoquinoniques, azoïques, xanthéniques, triarylméthaniques, aziniques, thiaziniques, phénothiaziniques, diaziniques, phénodiaziniques, acridiniques, cyanineméthiniques, azométhiniques, nitrés, phtalocyaniques, indoaniliques, indophénoliques, indoaminiques, et des colorants directs naturels.

Parmi les colorants directs benzéniques permettant de générer le groupe A selon l'invention, on peut citer de manière non limitative les composés suivants :
- 1,4-diamino-2-nitrobenzène,
- 1-amino-2 nitro-4-β-hydroxyéthylaminobenzène
- 1-amino-2 nitro-4-bis(β-hydroxyéthyl)-aminobenzène
- 1,4-bis(β -hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-hydroxy-2-amino-5-nitrobenzène
- 1-hydroxy-2-amino-4-nitrobenzène
- 1-hydroxy-3-nitro-4-aminobenzène
- 1-hydroxy-2-amino-4,6-dinitrobenzène
- 1-β-hydroxyéthyloxy-2-β-hydroxyéthylamino-5-nitrobenzène
- 1-méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-hydroxy-2-chloro-6-éthylamino-4-nitrobenzène
- 1-hydroxy-2-chloro-6-amino-4-nitrobenzène
- 1-hydroxy-6-bis-(β-hydroxyéthyl)-amino-3-nitrobenzène
- 1-β-hydroxyéthylamino-2-nitrobenzène
- 1-hydroxy-4-β-hydroxyéthylamino-3-nitrobenzène.

Parmi les colorants directs azoïques permettant de générer le groupe A selon l'invention, on peut citer les colorants azoïques cationiques décrits dans les demandes de brevets WO 95/15144, WO-95/01772 et EP-714954 dont le contenu fait partie intégrante de l'invention.

Parmi ces composés, on peut tout particulièrement citer les colorants suivants :
- chlorure de 1,3-diméthyl-2-[[4-(diméthylamino)phényl]azo]-1 H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
   ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-chloro-4'-hydroxy)phényl-acétylamino-6-méthoxy-1,4-benzoquinone imine
- 3-N(3'-chloro-4'-méthylamino)phényl-uréido-6-méthyl-1,4-benzoquinone imine
- 3-[4'-N-(éthyl,carbamylméthyl)-amino]-phényl-uréido-6-méthyl-1,4-benzoquinone imine

Parmi les colorants directs naturels permettant de générer le groupe A selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine.

Le groupe L représente un « bras de liaison », on entend par « bras de liaison » un atome ou un groupe d'atomes séparant le groupe chromophore du ou des groupements alcoxysilanes. C'est une chaîne hdyrocarbonée, pouvant être substituée ou interrompue par un hétéroatome, tel que par exemple O, N, ou Si, ou par un cycle aromatique ou hétéroaromatique pouvant être lui-même substitué.

Le bras de liaison peut être cationique ou non cationique.

Le bras de liaison est par exemple une chaîne hydrocarbonée en C₁-C₂₀, de préférence en C₁-C₆, linéaire ou ramifiée, un ou plusieurs des atomes de carbone de la chaîne pouvant être remplacés par un hétéroatome tel que le soufre, l'azote, l'oxygène, la chaîne hydrocarbonée pouvant être saturée ou insaturée, ou contenir un radical arylène ; un radical divalent téréphtalamide ; un radical divalent triazine ; un radical -NH-CO-.

La chaîne hydrocarbonée peut être substituée par exemple par un ou plusieurs radicaux hydroxy, alcoxy, amino, alkylamino, ou halogène.

A titre d'exemple de bras de liaison, on peut citer les radicaux alkylène (CₙH₂ₙ) comprenant de préférence de 1 à 6 atomes de carbone, par exemple méthylène, éthylène, propylène, etc ; les radicaux (hétéro)arylène par exemple phénylène ou naphtylène, phénanthrylène, triazinyle, pyrimidinyle, pyridinyle, pyridazinyle, quinoxalinyle, les radicaux alkyle-aryle-alkyle.

A titre de bras de liaison, on peut citer les triazines décrites dans le WO03/029359, les alkylènes cités dans US 5 708 151, les Alkyle-aryle-Alkyle cités dans US 5 708 151.

De manière préférée, le bras de liaison L est une chaîne alkylène en C₁-C₂₀, de préférence en C₁-C₆, linéaire ou ramifiée.

Dans le cadre des définitions des groupes R₄, R₅, R₆, par « substitué », on entend substitué par un groupement hydroxy ; un groupement halogène ; un groupement alcoxy en C₁-C₆ linéaire ou ramifié ; un groupement amino ; un groupement acétylamino ; un groupement alkylcarbonyle en C₁-C₆ linéaire ou ramifié ; un groupement hydroxycarbonyle ; un groupement alkoxycarbonyle en C₁₋C₆ linéaire ou ramifié.

Les composés azoïques de formule (I) et (II) peuvent être choisis parmi les composés suivants :
- benzensulfonate de 4-amino-5-[[4'-[(4-hydroxyphenyl)azo]-3,3'-dimethyl[1,1'-biphenyl]-4-yl]azo]-2-[[2-hydroxy-3-[3-(trimethoxysilyl)propoxy]propyl]amino]
- benzoate de 4-[(4-ethoxyphenyl)azo]-, 3-(triethoxysilyl)propyl ester
- benzoate de 4-[(4-ethoxyphenyl)azo]-, 2-(triethoxysilyl)ethyl ester
- acetamide, N-[2-[[4-amino-6-[(3,3,4,4,5,5,6,6,7,7,8,8,9,9,10,10-hexadecafluorodecyl)oxy]-1,3,5-triazin-2-yl]amino]-5-[[4,8-bis[[[3-(trimethoxysilyl)propyl]amino]sulfonyl]-2-naphthalenyl]azo]phenyl]
- acetamide, N-[2-[[4-amino-6-[(3,3,4,4,5,5,6,6,7,7,8,8,9,9,10,10,10-heptadecafluorodecyl)oxy]-1,3,5-triazin-2-yl]amino]-5-[[4,8-bis[[[3-(trimethoxysilyl)propyl]amino]sulfonyl]-2-naphthalenyl]azo]phenyl]
- acetamide, N-[2-[[4-amino-6-(2,2-difluoro-2-hydroxyethoxy)-1,3,5-triazin-2-yl]amino]-5-[[4,8-bis[[[3-(trimethoxysilyl)propyl]amino]sulfonyl]-2-naphthalenyl]azo]phenyl]
- acetamide, N-[2-[[4-amino-6-(2,2-difluoro-2-hydroxyethoxy)-1,3,5-triazin-2-yl]amino]-5-[[4,8-bis[[[3-(triethoxysilyl)propyl]amino]sulfonyl]-2-naphthalenyl]azo]phenyl]
- N-[4-[(1E)-phenylazo]phenyl]-N'-[3-(triethoxysilyl)propyl]urea
- carbamate de [3-(triethoxysilyl)propyl]-, 2-[ethyl[4-[(4-nitrophenyl)azo]phenyl]amino]ethyl ester
- carbamate de [3-(trimethoxysilyl)propyl]-, [[4-[(4-nitrophenyl)azo]phenyl]imino]di-2,1-ethanediyl ester
- carbamate de [3-(trimethoxysilyl)propyl]-, 2-[ethyl[4-[(4-nitrophenyl)azo]phenyl]amino]ethyl
- 2,7-Naphthalenedisulfonate de 4-amino-3-[[4-[[4-[[2-amino-4-[[[[3-(triethoxysilyl)propyl]amino]carbonyl]oxy]phenyl]azo]phenyl]amino]-3-sulfophenyl]azo]-5-hydroxy-6-(phenylazo)
- benzamide, 2-[(1E)-[4-(dimethylamino)phenyl]azo]-N-[3-(triethoxysilyl)propyl
- carbamate de [3-(triethoxysilyl)propyl]-, [[4-[(4-nitrophenyl)azo]phenyl]imino]di-2,1-ethanediyl ester
- 2-[ethyl[4-[(1E)-(4-nitrophenyl)azo]phenyl]amino]ethyl [3-(triethoxysilyl)propyl] carbamate
- benzonitrile, 4-[[4-[bis[2-[3-(trimethoxysilyl)propoxy]ethyl]amino]phenyl]azo]-,
- benzenesulfonamide, 4-(2-benzothiazolylazo)-N-[3-(trimethoxysilyl)propyl]
- 2-Propanol, 1,1'-[[[4-[(4-nitrophenyl)azo]phenyl]imino]bis(4,1-phenyleneimino)]bis[3-[3-(trimethoxysilyl)propoxy
- urea, N,N"-[(1Z)-azodi-4,1-phenylene]bis[N'-[3-(triethoxysilyl)propyl]
- urea, N,N"-[(1E)-azodi-4,1-phenylene]bis[N'-[3-(triethoxysilyl)propyl]
- iodure Pyridinium, 4-[6-[[4-[bis[2-[[(1,1-dimethylethyl)dimethylsilyl]oxy]ethyl]amino]phenyl]azo]-2-benzothiazolyl]-1-[3-(trimethoxysilyl)propyl]
- 2-Propenoate, 2-methyl-, 2-[ethyl[4-[(4-nitrophenyl)azo]phenyl]amino]ethyl ester
- carbamate de [3-(triethoxysilyl)propyl]-, 4-[(4-nitrophenyl)azo]phenyl ester,
- carbamate de [3-(triethoxysilyl)propyl]-, [[4-[[4-[(13,13-diethoxy-8-oxo-7,14-dioxa-9-aza-13-silahexadec-1-yl)sulfonyl]phenyl]azo]phenyl]imino]di-2,1-ethanediyl ester
- carbamate de [3-(triethoxysilyl)propyl]-, 6-[[4-[[4-[(9,9-diethoxy-4-oxo-3,10-dioxa-5-aza-9-siladodec-1-yl)methylamino]phenyl]azo]phenyl]sulfonyl]hexyl ester
- carbamate de [3-(triethoxysilyl)propyl]-, [[4-[[4-(butylsulfonyl)phenyl]azo]phenyl]imino]di-2,1-ethanediyl ester
- urea, N-[4-(phenylazo)phenyl]-N'-[3-(triethoxysilyl)propyl 1-[[4-[(4-nitrophenyl)azo]phenyl]amino]-3-[3-(trimethoxysilyl)propoxy]-2-propanol
- carbamate de [3-(triethoxysilyl)propyl]-, 2-[2,5-dimethyl-4-[(4-nitrophenyl)azo]phenoxy]ethyl ester
- pyridinium, 4-[[4-[bis[2-[[(1,1-dimethylethyl)dimethylsilyl]oxy]ethyl]amino]phenyl]azo]-1-[3-(trimethoxysilyl
- 2,7-Naphthalenedicarboxamide, 3-hydroxy-4-[[2-methoxy-5-[(phenylamino)carbonyl]phenyl]azo]-N2-1-naphthalenyl-N7-[2-[[3-(trimethoxysilyl)propyl] amino] ethyl]
- 2,7-Naphthalenedicarboxamide, 3-hydroxy-4-[[2-methoxy-5-[(phenylamino)carbonyl]phenyl]azo]-N2-1-naphthalenyl-N7-[3-(triethoxysilyl)propyl]
- 1H-Pyrazole-3-carboxamide, 4,5-dihydro-5-oxo-1-phenyl-4-(phenylazo)-N-[3-(trimethoxysilyl)propyl
- benzamide, 4-[[4-(dimethylamino)phenyl]azo]-N-[3-(triethoxysilyl)propyl]
- benzamide, 4-(phenylazo)-N-[3-(triethoxysilyl)propyl]
- benzamide, 2-[[4-(dimethylamino)phenyl]azo]-N-[3-(trimethoxysilyl)propyl]
- carbamate de [3-(triethoxysilyl)propyl]-, [[4-[(1E)-(4-nitrophenyl)azo]phenyl]imino]di-2,1-ethanediyl ester
- carbamate de [3-(triethoxysilyl)propyl]-, [[4-[[4-[[4-[[[[3-(triethoxysilyl)propyl]amino]carbonyl]amino]phenyl]sulfonyl]phenyl]a zo]phenyl]imino]di-2,1-ethanediyl ester
- 1H-Isoindole-5-carboxylic acid, 2,3-dihydro-1,3-dioxo-2-[3-(triethoxysilyl)propyl]-, 5-(diethylamino)-2-[[2-[[[2,3-dihydro-1,3-dioxo-2-[3-(triethoxysilyl)propyl]-1H-isoindol-5-yl]carbonyl]oxy]-4-nitrophenyl]azo]phenyl ester
- carbamate de [3-(triethoxysilyl)propyl]-, [[4-[[2-[(9,9-diethoxy-4-oxo-3,10-dioxa-5-aza-9-siladodec-1-yl)oxy]-4-nitrophenyl]azo]phenyl]imino]di-2,1-ethanediyl ester
- carbamate de [3-(triethoxysilyl)propyl]-, 2-[2-[[2-[(9,9-diethoxy-4-oxo-3,10-dioxa-5-aza-9-siladodec-1-yl)oxy]-4-(diethylamino)phenyl]azo]-5-nitrophenoxy]ethyl ester
- carbamate de [3-(triethoxysilyl)propyl]-, [ethyl[4-[(4-nitrophenyl)azo]phenyl]amino]methyl ester
- undecanamide, 11-[4-[(4-methylphenyl)azo]phenoxy]-N-[3-(triethoxysilyl)propyl]-,
- acetamide, N-[2-[[4,5-dicyano-1-[3-(trimethoxysilyl)propyl]-1H-imidazol-2-yl]azo]-5-(diethylamino)phenyl]
- benzamide, 4-[(5-cyano-1,6-dihydro-2-hydroxy-1,4-dimethyl-6-oxo-3-pyridinyl)azo]-N-[3-(trimethoxysilyl)propyl
- carbamate de [3-(triethoxysilyl)propyl]-, 6-[methyl[4-[(4-nitrophenyl)azo]phenyl]amino]hexyl ester
- undecanamide, 11-[4-[(4-pentylphenyl)azo]phenoxy]-N-[11-(triethoxysilyl)undecyl]
- hexanamide, 6-[4-[(4-pentylphenyl)azo]phenoxy]-N-[11-(triethoxysilyl)undecyl]
- propanamide, 3-[4-[(4-pentylphenyl)azo]phenoxy]-N-[11-(triethoxysilyl)undecyl
- undecanamide, 11-[4-(phenylazo)phenoxy]-N-[11-(triethoxysilyl)undecyl]
- hexanamide, 6-[4-(phenylazo)phenoxy]-N-[11-(triethoxysilyl)undecyl]
- propanamide, 3-[4-(phenylazo)phenoxy]-N-[11-(triethoxysilyl)undecyl]
- propanamide, 3-[4-[(4-pentylphenyl)azo]phenoxy]-N-[3-(triethoxysilyl)propyl]
- undecanamide, 11-[4-(phenylazo)phenoxy]-N-[3-(triethoxysilyl)propyl]
- propanamide, 3-[4-(phenylazo)phenoxy]-N-[3-(triethoxysilyl)propyl
- hexanamide, 6-[4-[(4-pentylphenyl)azo]phenoxy]-N-[3-(triethoxysilyl)propyl]-,
- carbamate de [3-(triethoxysilyl)propyl]-, [[3-methyl-4-[(4-nitrophenyl)azo]phenyl]imino]di-2,1-ethanediyl ester
- hexanamide, 6-[5-[(4-cyanophenyl)azo]-2-(hexyloxy)phenoxy]-N-[3-(triethoxysilyl)propyl]-,
- hexanamide, 6-[2-[(4-cyanophenyl)azo]-5-(hexyloxy)phenoxy]-N-[3-(triethoxysilyl)propyl
- acetamide, 2-[5-(hexyloxy)-2-[(4-hexylphenyl)azo]phenoxy]-N-[3-(triethoxysilyl)propyl]-,
- hexanamide, 6-[5-(hexyloxy)-2-[(4-hexylphenyl)azo]phenoxy]-N-[3-(triethoxysilyl)propyl]-,
- hexanamide, 6-[4-[(4-cyanophenyl)azo]phenoxy]-N-[3-(triethoxysilyl)propyl]
- undecanamide, 11-[4-[(4-pentylphenyl)azo]phenoxy]-N-[3-(triethoxysilyl)propyl]
- hexanamide, 6-[4-[(4-pentylphenyl)azo]phenoxy]-N-[3-(triethoxysilyl)propyl]
- benzamide, 3-[[3-hydroxy-4-(2-pyridinylazo)-2-naphthalenyl]azo]-N-[3-(triethoxysilyl)propyl]
- benzamide, 4-[(4-methoxyphenyl)azo]-N-[3-(triethoxysilyl)propyl]-,
- hexanamide, 6-[4-(phenylazo)phenoxy]-N-[3-(triethoxysilyl)propyl]-,
- benzamide, 4-[[4-(hexyloxy)phenyl]azo]-N-[3-(triethoxysilyl)propyl]-,
- carbamate de [3-(triethoxysilyl)propyl]-, [[3-chloro-4-[(4-nitrophenyl)azo]phenyl]imino]di-2,1-ethanediyl ester
- carbamate de [3-(triethoxysilyl)propyl]-, [[4-[(2,6-dichloro-4-nitrophenyl)azo]phenyl]imino]di-2,1-ethanediyl ester
- carbamate de [3-(triethoxysilyl)propyl]-, [[4-[[4-[(9,9-diethoxy-4-oxo-3,10-dioxa-5-aza-9-siladodec-1-yl)sulfonyl]phenyl]azo]phenyl]imino]di-2,1-ethanediyl ester
- carbamate de [3-(triethoxysilyl)propyl]-, 2-methyl-4-[[4-(phenylazo)phenyl]azo]phenyl ester
- carbamate de [3-(triethoxysilyl)propyl]-, 2-[[4-[(2-chloro-4-nitrophenyl)azo]phenyl]ethylamino]ethyl ester
- carbamate de [3-(triethoxysilyl)propyl]-, 4-[(4-nitrophenyl)azo]-1,3-phenylene ester
- hexanamide, 6-[4-[(4-hexylphenyl)azo]phenoxy]-N-[3-(triethoxysilyl)propyl]-,
- 2,7-Naphthalenedisulfonic acid, 4-hydroxy-5-[[4-[(3-sulfophenyl)amino]-6-[[2-[3-(triethoxysilyl)propoxy]ethyl]amino]-1,3,5-triazin-2-yl]amino]-3-[[4-[[2-[[2-[3-(triethoxysilyl)propoxy]ethyl]amino]ethyl]sulfonyl]phenyl]azo]
- urea, N-[4-[(4-nitrophenyl)azo]phenyl]-N'-[3-(triethoxysilyl)propyl]-,
- benzoate de 2-[[4-(dimethylamino)phenyl]azo]-, 3-(triethoxysilyl)propyl ester
- acetamide, N-[3-(triethoxysilyl)propyl]-2-[4-[[4-[[[3-(triethoxysilyl)propyl]amino]methoxy]phenyl]azo]phenoxy
- alanine, N-[3-(triethoxysilyl)propyl]-, 4-[(4-methoxyphenyl)azo]phenyl ester
- acetamide, 2-[4-[(4-methoxyphenyl)azo]phenoxy]-N-[3-(triethoxysilyl)propyl]
- diazene, (4-nitrophenyl)[4-[3-(trimethoxysilyl)propoxy]phenyl]
- hexanamide, 6-[4-[(4-ethylphenyl)azo]phenoxy]-N-[3-(triethoxysilyl)propyl
- undecanamide, 11-[5-(hexyloxy)-2-[(4-hexylphenyl)azo]phenoxy]-N-[3-(triethoxysilyl)propyl]
- cyclohexanecarboxamide, 4-[4-[[4-(pentyloxy)phenyl]azo]phenyl]-N-[3-(triethoxysilyl)propyl]
- benzeneheptanamide, 4-[[4-(pentyloxy)phenyl]azo]-N-[3-(triethoxysilyl)propyl]
- hexanamide, 6-[4-[(4-cyanophenyl)azo]phenoxy]-N-[3-(triethoxysilyl)propyl]
- acetamide, 2-[4-[(4-hexylphenyl)azo]phenoxy]-N-[3-(triethoxysilyl)propyl
- propanamide, 3-[4-[(4-hexylphenyl)azo]phenoxy]-N-[3-(triethoxysilyl)propyl]
- butanamide, 4-[4-[(4-hexylphenyl)azo]phenoxy]-N-[3-(triethoxysilyl)propyl]
- benzamide, 4-[[4-(hexyloxy)phenyl]azo]-N-[3-(triethoxysilyl)propyl
- benzamide, 4-[(4-methoxyphenyl)azo]-N-[3-(triethoxysilyl)propyl]
- hexanamide, 6-[4-(phenylazo)phenoxy]-N-[3-(triethoxysilyl)propyl]
- hexanamide, 6-[4-[(4-chlorophenyl)azo]phenoxy]-N-[3-(triethoxysilyl)propyl]
- hexanamide, 6-[4-[(4-cyclohexylphenyl)azo]phenoxy]-N-[3-(triethoxysilyl)propyl
- benzamide, 4-[(8-hydroxy-5-quinolinyl)azo]-N-[3-(triethoxysilyl)propyl
- benzensulfonate de 4-amino-5-[[4'-[(4-hydroxyphenyl)azo]-3,3'-dimethyl[1,1'-biphenyl]-4-yl]azo]-2-[[2-hydroxy-3-[3-(trimethoxysilyl)propoxy]propyl]amino]-, monosodium salt
- phenol, 5-[[4-chloro-6-[[3-(triethoxysilyl)propyl]amino]-1,3,5-triazin-2-yl]oxy]-2-[(2-hydroxyphenyl)azo
- 1,3-Naphthalenedisulfonic acid, 6-[[4-[[2-hydroxy-3-[3-(triethoxysilyl)propoxy]propyl]amino]phenyl]azo]
- 1,3-Naphthalenedisulfonic acid, 6-[[4-[[4-[[2-hydroxy-3-[3-(triethoxysilyl)propoxy]propyl]amino]phenyl]azo]-1-naphthalenyl]azo]
- benzoate de 2-[[2-hydroxy-3-[3-(triethoxysilyl)propoxy]propyl]amino]-5-[(4-nitrophenyl)azo]
- 2-Propanol, 1-[[4-[(4-methyl-2-nitrophenyl)azo]phenyl]amino]-3-[3-(triethoxysilyl)propoxy]
- 2-Propanol, 1-[[4-[(2-chlorophenyl)azo]phenyl]amino]-3-[3-(triethoxysilyl)propoxy]
- 2-Propanol, 1-[[4-[(2-methylphenyl)azo]phenyl]amino]-3-[3-(triethoxysilyl)propoxy]
- 2-Propanol, 1-[[4-[(4-nitrophenyl)azo]phenyl]amino]-3-[3-(triethoxysilyl)propoxy]
- carbamate de [3-(trimethoxysilyl)propyl]-, 4-[(4-nitrophenyl)azo]phenyl ester
- carbamate de [3-(trimethoxysilyl)propyl]-, 4-(phenylazo)-1-naphthalenyl ester
- carbamate de [3-(trimethoxysilyl)propyl]-, 4-(phenylazo)phenyl ester
- urea, N-[4-(phenylazo)phenyl]-N'-[3-(trimethoxysilyl)propyl]
- urea, N-[4-[[4-(dimethylamino)phenyl]azo]phenyl]-N'-[3-(trimethoxysilyl)propyl]
- 2-Propanol, 1-[[4-(phenylazo)phenyl]amino]-3-[3-(trimethoxysilyl)propoxy]
- 2-Propanol, 1-[[4-(phenylazo)phenyl]amino]-3-[3-(triethoxysilyl)propoxy]
- 2-Propanol, 1-[[4-[(2,4-dinitrophenyl)azo]phenyl]amino]-3-[3-(triethoxysilyl)propoxy]
- 2,7-Naphthalenedisulfonic acid, 4-hydroxy-5-[[p-[methyl[3-(triethoxysilyl)propyl]amino]phenyl]azo]
- 1,3,6-Naphthalenetrisulfonic acid, 8-[[p-[methyl[3-(triethoxysilyl)propyl] amino]phenyl] azo]
- s-Triazine, 2-chloro-4-[4-(p-tolylazo)-o-toluidino]-6-[[3-(triethoxysilyl)propyl] amino]
- 2-Naphthol, 1-[[p-[2-(triethoxysilyl)ethyl]phenyl]azo]
- benzensulfonate de p-[[p-[methyl[3-(triethoxysilyl)propyl]amino]phenyl]azo
- 1,3-Naphthalenedisulfonic acid, 7-[[p-[methyl[3-(triethoxysilyl)propyl]amino]phenyl]azo]
- s-Triazine, 2-anilino-4-(4-p-tolylazo-o-toluidino)-6-[[3-(triethoxysilyl)propyl] amino]
- benzoate de p-[[p-[methyl[3-(triethoxysilyl)propyl]amino]phenyl]azo]
- benzoate de o-[[p-[methyl[3-(triethoxysilyl)propyl]amino]phenyl]azo]
- aniline, N-methyl-p-[(p-nitrophenyl)azo]-N-[3-(triethoxysilyl)propyl]
- aniline, N-methyl-p-(phenylazo)-N-[3-(triethoxysilyl)propyl].

Les composés de formule (I) et (II) peuvent également être choisis parmi les composés suivants :
- xanthylium, 3,6-bis(diethylamino)-9-[2-(ethoxycarbonyl)-4-[[[3-(triethoxysilyl)propyl]amino]sulfonyl]phenyl]-
- benzo[a]phenoxazin-7-ium, 9-(dimethylamino)-2-hydroxy-5-[[3-(triethoxysilyl)propyl]amino]-
- 1H,5H,11H,15H-Xantheno[2,3,4-ij:5,6,7-i'j']diquinolizin-18-ium, 9-[2-(ethoxycarbonyl)-4-[[[3-(triethoxysilyl)propyl]amino]sulfonyl]phenyl]-2,3,6,7,12,13,16,17-octahydro
- pyrano[3,2-g:5,6-g']diquinolin-13-ium, 6-(2-carboxy-3,4,5,6-tetrachlorophenyl)-1-ethyl-1,2,10,11-tetrahydro-2,2,4,8,10,10-hexamethyl-11-[4-oxo-4-[[3-(triethoxysilyl)propyl]amino]butyl]
- chlorure de 1H,5H,11H,15H-Xantheno[2,3,4-ij:5,6,7-i'j']diquinolizin-18-ium, 9-[2-(ethoxycarbonyl)-4-[[[3-(triethoxysilyl)propyl]amino]sulfonyl]phenyl]-2,3,6,7,12,13,16,17-octahydro-
- chlorure de Xanthylium, 3,6-bis(diethylamino)-9-[2-(ethoxycarbonyl)-4-[[[3-(triethoxysilyl)propyl]amino]sulfonyl]phenyl]
- sel interne de 1H,5H,11H,15H-Xantheno[2,3,4-ij:5,6,7-i'j']diquinolizin-18-ium, 9-[2-carboxy-4-[[[3-(triethoxysilyl)propyl]amino]sulfonyl]phenyl]-2,3,6,7,12,13,16,17-octahydro
- sel interne de Xanthylium, 9-[2-carboxy-4-[[[3-(triethoxysilyl)propyl]amino]sulfonyl]phenyl]-3,6-bis(diethylamino)
- sel interne de 1H,5H,11H,15H-Xantheno[2,3,4-ij:5,6,7-i'j']diquinolizin-18-ium, 2,3,6,7,12,13,16,17-octahydro-9-[2-sulfo-4-[[[3-(triethoxysilyl)propyl]amino]sulfonyl]phenyl]
- sel interne de Xanthylium, 3,6-bis(diethylamino)-9-[2-sulfo-4-[[[3-(triethoxysilyl)propyl]amino]sulfonyl]phenyl]
- bromure de Phenothiazin-5-ium, 3,7-bis[bis[3-(trimethoxysilyl)propyl]amino]-, bromide
- benzo[a]phenoxazin-7-ium, 9-(dimethylamino)-5-[[3-(triethoxysilyl)propyl]amino]-
- chlorure de Benzo[a]phenoxazin-7-ium, 9-(dimethylamino)-2-hydroxy-5-[[3-(triethoxysilyl)propyl]amino]
- chlorure de Benzo[a]phenoxazin-7-ium, 9-(dimethylamino)-5-[[3-(triethoxysilyl)propyl]amino]
- sel interne de Xanthylium, 3,6-bis(diethylamino)-9-[2-sulfo-4-[[[3-(diethoxymethylsilyl)propyl]amino]sulfonyl]phenyl].

Les composés de formule (I) et (II) peuvent également être choisis parmi les composés suivants :
- 2-naphthalenecarboxamide, 4-[[4-(diethylamino)-2-methylphenyl]imino]-1,4-dihydro-1-oxo-N-[3-(trimethoxysilyl)propyl]
- carbamate de [3-(triethoxysilyl)propyl]-, 2-[[4-[[5-(acetylamino)-2-methyl-4-oxo-2,5-cyclohexadien-1-ylidene]amino]-3-methylphenyl]ethylamino]ethyl ester
- 2-Naphthalenecarbonitrile, 8-amino-5-[(4-heptylphenyl)amino]-1,4-dihydro-1,4-dioxo-3-[[3-(triethoxysilyl)propyl]amino]
- 1H-Naphth[2,3-f]isoindole-1,3,5,10(2H)-tetrone, 4,11-diamino-2-[3- (diethoxymethylsilyl)propyl]-
- 1H-Pyrrole-2,5-dione, 1-[3-(diethoxymethylsilyl)propyl]-3-[4-(dimethylamino)phenyl]-4-[(4-methylphenyl)sulfonyl]-
- 1 H-Pyrrole-2,5-dione, 3-[4-(dimethylamino)phenyl]-4-[(4-methylphenyl)sulfonyl]-1-[3-(triethoxysilyl)propyl]-
- 2-Naphthalenecarbonitrile, 8-amino-5-[[4-(dimethylamino)phenyl]amino]-1,4-dihydro-1,4-dioxo-3-[[3-(triethoxysilyl)propyl] amino]-
- 2-Naphthalenecarbonitrile, 8-amino-1,4-dihydro-5-[(4-methoxyphenyl)amino]-1,4-dioxo-3-[[3-(triethoxysilyl)propyl]amino]-
- 2-Naphthalenecarbonitrile, 8-amino-1,4-dihydro-5-[(4-methylphenyl)amino]-1,4-dioxo-3-[[3-(triethoxysilyl)propyl]amino]-
- 2-Naphthalenecarbonitrile, 8-amino-5-[(4-chlorophenyl)amino]-1,4-dihydro-1,4-dioxo-3-[[3-(triethoxysilyl)propyl] amino]-
- 2-Naphthalenecarbonitrile, 8-amino-3-[[3-(diethoxymethylsilyl)propyl]amino]-1,4-dihydro-1,4-dioxo-
- 2-Naphthalenecarbonitrile, 8-amino-1,4-dihydro-1,4-dioxo-5-(phenylamino)-3-[[3-(triethoxysilyl)propyl]amino]-
- 1H-Naphth[2,3-f]isoindole-1,3,5,10(2H)-tetrone, 4,11-diamino-2-[3-(triethoxysilyl)propyl]-
- 2-Naphthalenecarbonitrile, 8-amino-1,4-dihydro-1,4-dioxo-3-[[3-(triethoxysilyl)propyl]amino]-
- dibenzo[a,c]phenazinium, 9-phenyl-11-[[3-(triethoxysilyl)propyl]amino]-,chlorure.

Les composés de formule (I) peuvent également être choisis parmi les composés suivants :
- benzamide, N-[3-(dimethoxymethylsilyl)propyl]-4-[(8-hydroxy-5-quinolinyl)azo]
- benzenamine, N-[3-(diethoxymethylsilyl)propyl]-N-methyl-4-[(4-nitrophenyl)azo
- 2-Propenoate de 2-methyl-, 3-[bis[2-[ethyl[4-[(4-nitrophenyl)azo]phenyl]amino]ethoxy]methylsilyl]propyl ester
- benzamide, N-cyclohexyl-3-[(2,2-dicyanoethyl)azo]-N-[3-(dimethoxymethylsilyl)propyl]
- 2-Propenoate de 2-methyl-, 2-[ethyl[4-[(4-nitrophenyl)azo]phenyl]amino]ethyl ester
- 2-[2-[[(2-bicyclo[4.2.0]octa-1,3,5-trien-3-ylethenyl)oxy][(2-bicyclo[4.2.0]oct-3-ylethenyl)oxy]methylsilyl]ethyl]-N,N-dimethyl-4-[(4-nitrophenyl)azo]
- hexanamide, N-[3-(diethoxymethylsilyl)propyl]-6-[4-[(4-hexylphenyl)azo]phenoxy]
- hexanamide, N-[3-(diethoxymethylsilyl)propyl]-6-[4-[(4-hexylphenyl)azo]phenoxy

La composition selon la présente invention peut comprendre de 0,001 à 20%, de préférence de 0,01 à 10%, et de manière encore préférée de 0,1 à 5% en poids de colorant(s) direct(s) de formule (I) par rapport au poids total de la composition.

Les colorants de l'invention peuvent être préparés selon des réactions chimiques connues en soi à partir de chromophores fonctionnalisés capables de réagir avec le bras de liaison choisi. Les colorants peuvent notamment être préparés par le procédé décrit dans le document FR 2 421 934.

La composition tinctoriale conforme à l'invention peut en outre contenir un ou plusieurs colorants directs additionnels autres que les colorants directs de formule (I) décrits ci-dessus, pouvant notamment être choisis parmi les colorants directs mentionnés plus haut, et notamment parmi les colorants nitrés de la série benzénique, neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Le ou les colorants directs additionnels représentent de préférence de 0,001 à 20% en poids du poids total de la composition prête à l'emploi et encore plus préférentiellement de 0,005 à 10% en poids.

Les compositions de l'invention peuvent également comprendre un ou plusieurs précurseurs de colorants d'oxydation, et notamment des bases d'oxydation.

A titre d'exemple, les bases d'oxydation présentes dans les composition selon l'invention sont choisies parmi les phénylènediamines, les bis-phénylalkylènediamines, les para-aminophénols, les ortho-aminophénols, les bases hétérocycliques autres que les paraphénylènediamines hétérocycliques de formule (I) et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ;
ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition.

La ou les bases d'oxydation présentes dans la composition de l'invention sont en général présentes en quantité comprise allant de 0,001 à 20 % en poids environ du poids total de la composition tinctoriale, de préférence allant de 0,005 à 6 %.

La composition de la présente invention peut en outre comprendre un ou plusieurs précurseurs de colorant d'oxydation choisis parmi les coupleurs.

Les coupleurs peuvent être choisis parmi les coupleurs conventionnellement utilisés pour la teinture de fibres kératiniques. Parmi ces coupleurs, on peut notamment citer les métaphénylènediamines, les métadiphénols, les coupleurs naphtaléniques, les coupleurs hétérocycliques ainsi que leur sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Dans la composition de la présente invention, le ou les coupleurs sont généralement présents en une quantité allant de 0,001 à 20 % en poids du poids total de la composition tinctoriale, de préférence allant de 0,005 à 6 %.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

Le milieu approprié pour la teinture appelé aussi support de teinture est un milieu cosmétique généralement constitué par de l'eau ou par un mélange d'eau et/ou d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 99 % en poids environ par rapport au poids total de la composition tinctoriale, et encore plus préférentiellement entre 10 et 95 % en poids environ.

La composition tinctoriale conforme à l'invention peut également renfermer divers adjuvants utilisés classiquement dans les compositions pour la teinture des cheveux, tels que des agents tensioactifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 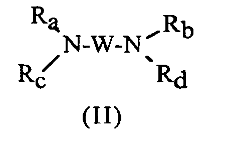 dans laquelle W est un reste propylène éventuellement substitué par un groupement hydroxyle ou un radical alkyle en C₁-C₄ ; Ra, Rb, Rc et Rd, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

La mise en oeuvre du procédé selon la présente demande peut se traduire par l'application d'une composition tinctoriale selon la présente demande sur les fibres kératiniques, puis une étape consistant à laisser poser pendant une période suffisante pour permettre la coloration des cheveux. Cette période est généralement comprise entre 5 minutes et 1 heure et de préférence entre 15 minutes et 1 heure. Cette coloration directe peut s'effectuer en présence d'un agent oxydant.

Un autre procédé de teinture des fibres kératiniques peut consister à appliquer sur les fibres kératiniques une première composition telle que définie ci-dessus contenant au moins un composé de formule (I), à laisser poser pendant une période comprise entre 5 minutes et une heure, puis après un éventuel rinçage, à appliquer sur les fibres kératiniques une composition contenant au moins un agent alcalin tel que défini ci-dessus, ceci pendant un temps de pose compris entre quelques secondes et 30 mn

Lorsque la composition selon l'invention comprend au moins un précurseur de colorant d'oxydation, le procédé de teinture des fibres kératiniques peut comprendre une étape dans laquelle la couleur est révélée à l'aide d'un agent oxydant. La couleur peut être révélée à pH acide, neutre ou alcalin et l'agent oxydant peut être ajouté à la composition de l'invention juste au moment de l'emploi ou il peut être mis en oeuvre à partir d'une composition oxydante le contenant, appliqué simultanément ou séquentiellement à la composition de l'invention.

Le procédé peut ainsi comprendre l'application d'une composition selon l'invention comprenant au moins un précurseur de colorant d'oxydation, puis une étape consistant à laisser poser pendant une durée comprise entre 5 et 60 minutes, puis après un éventuel rinçage l'application d'une composition contenant au moins un agent oxydant et au moins un agent alcalin qu'on laisse poser de 5 minutes à 60 minutes.

Selon un mode de réalisation particulier, la composition selon la présente invention comprenant au moins un composé de formule (I), au moins un précurseur de colorant d'oxydation est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 5 minutes à 1 heure environ, de préférence 15 minutes à 1 heure environ, les fibres kératiniques sont rincées, éventuellement lavées au shampooing, puis rincées à nouveau, et séchées.

Selon un autre mode de réalisation, la composition comprenant au moins un composé de formule (I) et un précurseur de colorant d'oxydation est appliquée sur les fibres kératiniques, puis après un temps de pose compris entre 5 et 60 mn et après un éventuel rinçage on applique une composition contenant au moins un agent oxydant et au moins un agent alcalin. Après un nouveau temps de pose compris entre 5 mn et une heure environ, de préférence compris entre 15 minutes et une heure, les fibres kératiniques sont rincées, lavées éventuellement au shampooing puis rincées à nouveau, puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et de préférence entre 5 et 11 environ et de manière encore plus préférée entre 6 et 10,5. Il peut être ajusté à la valeur désirée au moyen d'agents régulateurs de pH, acidifiants ou alcalinisants habituellement utilisés en teinture des fibres kératiniques et tels que définis précédemment.

La composition appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

La présente demande a également pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent sont destinés à illustrer l'invention, sans toutefois présenter un caractère limitatif.

**Exemples de composition selon l'invention :**

| | Composition A | Composition B |
|---|---|---|
| ethanol | 89g | - |
| eau | 10g | 97g |
| monoéthanolamine | - | 3 g |
| colorant | 1g | - |

Deux colorants à fonction trialcoxysilane ont été utilisés :
- le 3-(2,4-dinitrophénylamino)-propyltriéthoxysilane (composé de formule X), et
- le N-(triéthoxysilylpropyl)-dansylamide (composé de formule Y)

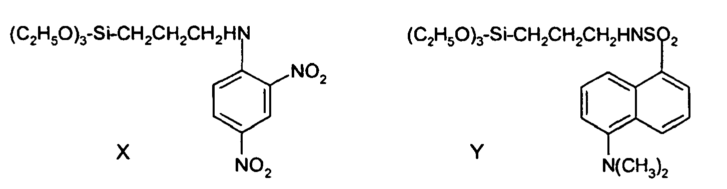

La composition A est appliquée sur des mèches de cheveux blancs naturels et blancs permanentés de 1g avec un rapport de bain équivalent à 10g de composition A pour 1g de cheveux.

Après 30 minutes de pose, on applique la composition B (rapport de bain de 5g de composition B pour 1 g de cheveux).

Après 15 minutes de pose, les mèches sont rincées et lavées au shampooing, puis séchées. Durant la totalité du temps de pose, les mèches sont placées à 45°C.

### Résultats

Le reflet obtenu lorsque la composition A comprend le colorant X est un doré intense. Il est très résistant vis-à-vis de lavages répétés.

Lorsque la composition A comprend le colorant Y, le reflet obtenu est un jaune intense qui est très résistant vis-à-vis de lavages répétés.

## Revendications

1. Utilisation à titre de colorant direct dans des compositions tinctoriales pour fibres kératiniques, en particulier les fibres kératiniques humaines telles que les cheveux, ou pour la fabrication de telles compositions, d'un composé de type organosilane de formule (I) : 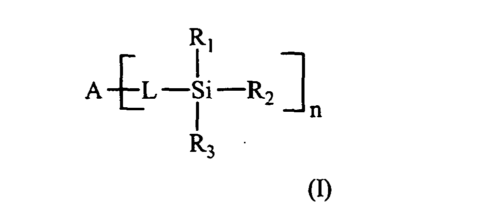 dans laquelle :
le groupe A est un groupe à fonction colorante directe,
le groupe L est un bras de liaison,
R₁, R₂, et R₃, indépendamment les uns des autres, représentent un hydrogène ; un radical mono ou polyhydroxyalkyle ayant de 1 à 10 atomes de carbone ; un radical mono ou polyaminoalkyle ayant de 1 à 10 atomes de carbone ; un radical mono ou polyhalogènealkyle ayant de 1 à 10 atomes de carbone ; un radical mono ou polyalcoxyalkyle, le radical alcoxy étant en C₁-C₁₀ et le radical alkyle en C₁-C₁₀ ; un radical aryle en C₆-C₁₈ ; un radical mono ou polyaminoaryle en C₆-C₁₈ ; un radical mono ou polyhydroxyaryle en C₆-C₁₈ ; un radical mono ou polyalcoxyaryle, le radical alcoxy étant en C₁-C₁₀ et le radical aryle en C₆-C₁₈ ; un radical alkylaryle, le radical alkyle étant en C₁-C₁₀ et le radical aryle en C₆-C₁₈ ; un radical arylalkyle, le radical alkyle étant en C₁-C₁₀ et le radical aryle en C₆-C₁₈ ; un radical carboxyalkyle ou sulfoalkyle, le radical alkyle étant en C₁-C₁₀,
n est un entier compris entre 1 et 10.

2. Utilisation selon la revendication 1, **caractérisée en ce que** le composé de type organosilane est de formule (II) : 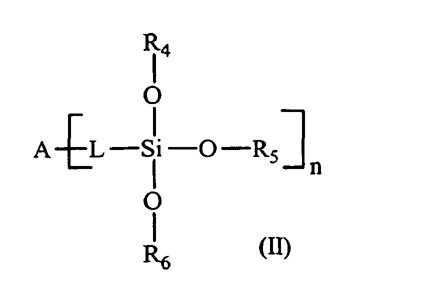 dans laquelle :
le groupe A est un groupe à fonction colorante directe,
le groupe L est un bras de liaison,
R₄, R₅, et R₆, indépendamment les uns des autres, représentent un radical alkyle en C₁-C₁₀, de préférence en C₁-C₆, linéaire ou ramifié,
n est un entier compris entre 1 et 10.

3. Utilisation selon la revendication 1 ou 2, **caractérisée en ce que** le groupe A est choisi parmi les radicaux issus des colorants nitro aromatiques, anthraquinoniques, naphtoquinoniques, benzoquinoniques, azoïques, xanthéniques, triarylméthaniques, aziniques, thiaziniques, phénothiaziniques, diaziniques, phénodiaziniques, acridiniques, cyanineméthiniques, azométhiniques, nitrés, phtalocyaniques, indoaniliques, et des colorants directs naturels.

4. Utilisation selon l'une des revendications 1 à 3, **caractérisée en ce que** le bras de liaison L est une chaîne hydrocarbonée en C₁-C₂₀, de préférence en C₁-C₆, linéaire ou ramifiée, un ou plusieurs atomes de carbone de la chaîne pouvant être remplacés par un hétéroatome, la chaîne pouvant être saturée ou insaturée, ou contenir un radical arylène, un radical divalent téréphtalamide, un radical divalent triazine, un radical NHCO.

5. Utilisation selon la revendication 4, **caractérisée en ce que** la chaîne hydrocarbonée L est substituée par un plusieurs radicaux hydroxy, alcoxy, amino, alkylamino, ou halogène.

6. Utilisation selon l'une des revendications 4 ou 5, **caractérisée en ce que** le bras de liaison L est une chaîne alkylène en C₁-C₂₀, de préférence en C₁-C₆, linéaire ou ramifiée.

7. Composition tinctoriale pour la teinture des fibres kératiniques, en particulier des fibres kératiniques humaines telles que les cheveux comprenant dans un milieu de teinture approprié au moins une base d'oxydation et au moins un composé de type organosilane de formule (I) : 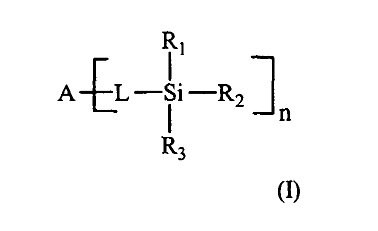 dans laquelle :
le groupe A est un groupe à fonction colorante directe,
le groupe L est un bras de liaison,
R₁, R₂, et R₃, indépendamment les uns des autres, représentent un hydrogène ; un radical mono ou polyhydroxyalkyle ayant de 1 à 10 atomes de carbone ; un radical mono ou polyaminoalkyle ayant de 1 à 10 atomes de carbone ; un radical mono ou polyhalogènealkyle ayant de 1 à 10 atomes de carbone ; un radical mono ou polyalcoxyalkyle, le radical alcoxy étant en C₁-C₁₀ et le radical alkyle en C₁-C₁₀ ; un radical aryle en C₆-C₁₈ ; un radical mono ou polyaminoaryle en C₆-C₁₈ ; un radical mono ou polyhydroxyaryle en C₆-C₁₈ ; un radical mono ou polyalcoxyaryle, le radical alcoxy étant en C₁-C₁₀ et le radical aryle en C₆-C₁₈ ; un radical alkylaryle, le radical alkyle étant en C₁-C₁₀ et le radical aryle en C₆-C₁₈ ; un radical arylalkyle, le radical alkyle étant en C₁-C₁₀ et le radical aryle en C₆-C₁₈ ; un radical carboxyalkyle ou sulfoalkyle, le radical alkyle étant en C₁-C₁₀,
n est un entier compris entre 1 et 10.

8. Composition selon la revendication 7, **caractérisée en ce que** le composé de type organosilane est de formule (II) : 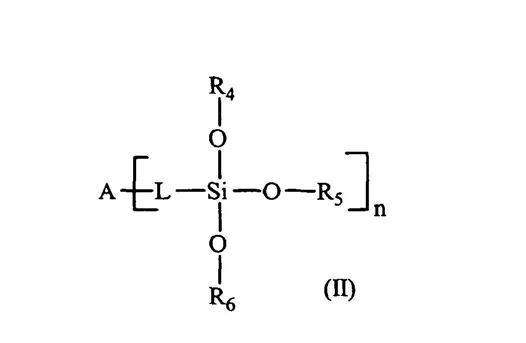 dans laquelle :
le groupe A est un groupe à fonction colorante directe,
le groupe L est un bras de liaison,
R₄, R₅, et R₆, indépendamment les uns des autres, représentent un radical alkyle en C₁-C₁₀, de préférence en C₁-C₆, linéaire ou ramifié.
n est un entier compris entre 1 et 10.

9. Composition selon la revendication 7 ou 8, **caractérisée en ce que** le groupe A est choisi parmi les radicaux issus des colorants nitro aromatiques, anthraquinoniques, naphtoquinoniques, benzoquinoniques, azoïques, xanthéniques, triarylméthaniques, aziniques, thiaziniques, phénothiaziniques, diaziniques, phénodiaziniques, acridiniques, cyanineméthiniques, azométhiniques, nitrés, phtalocyaniques, indoaniliques, et des colorants directs naturels.

10. Composition selon l'une des revendications 7 à 9, **caractérisée en ce que** le bras de liaison L est une chaîne hydrocarbonée en C₁-C₂₀, de préférence en C₁-C₆, linéaire ou ramifiée, un ou plusieurs atomes de carbone de la chaîne pouvant être remplacés par un hétéroatome, la chaîne pouvant être saturée ou insaturée, ou contenir un radical arylène, un radical divalent téréphtalamide, un radical divalent triazine, un radical NHCO.

11. Composition selon la revendications 10, **caractérisée en ce que** la chaîne hydrocarbonée L est substituée par un plusieurs radicaux hydroxy, alcoxy, amino, alkylamino, ou halogène.

12. Composition selon l'une des revendications 10 et 11, **caractérisée en ce que** le bras de liaison est une chaîne alkylène en C₁-C₂₀, de préférence en C₁-C₆, linéaire ou ramifiée.

13. Composition selon l'une des revendications 7 à 12, **caractérisée en ce qu'**elle comprend de 0,001 à 20%, de préférence de 0,01 à 10%, et de manière encore préférée de 0,1 à 5% en poids de colorant(s) direct(s) de formule (I) par rapport au poids total de la composition.

14. Composition selon l'une des revendications 7 à 13, **caractérisée en ce qu'**elle comprend au moins un précurseur de colorant d'oxydation choisi parmi les bases d'oxydation.

15. Composition selon la revendication 14, **caractérisée en ce que** la base d'oxydation est choisie parmi les paraphénylènediamines, les bis-phénylalkylènediamines, les paraaminophénols, les orthoaminophénols, les bases hétérocycliques et leurs sels d'addition.

16. Composition tinctoriale selon l'une des revendications 14 et 15, **caractérisée en ce que** la ou les bases d'oxydation sont présentes en une quantité comprise entre 0,001 à 20 % en poids et de préférence entre 0,005 et 6 % en poids par rapport au poids total de la composition.

17. Composition selon l'une des revendications 7 à 16, **caractérisée en ce qu'**elle contient au moins un précurseur de colorant d'oxydation choisi parmi les coupleurs.

18. Composition selon la revendication 17, **caractérisée en ce que** le coupleur est choisi parmi les métaphénylènediamines, les métaaminophénols, les métadiphénols, les coupleurs naphtaléniques, les coupleurs hétérocycliques et leurs sels d'addition.

19. Composition selon la revendication 18, **caractérisée en ce que** le coupleur est choisi parmi le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino, 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

20. Composition selon l'une des revendications 17 à 19, **caractérisée en ce que** le ou les coupleurs sont présents en une quantité comprise entre 0,001 et 20 % de préférence entre 0,005 et 6 % en poids par rapport au poids total de la composition.

21. Composition selon l'une des revendications 7 à 20, **caractérisée en ce qu'**elle comprend au moins un colorant direct additionnel.

22. Composition selon l'une des revendications 7 à 21, **caractérisée en ce qu'**elle comprend au moins un solvant organique.

23. Composition selon la revendication 22, **caractérisé en ce que** le solvant organique est choisi parmi l'éthanol, le propylène glycol, le glycérol, et les monoéthers de polyols.

24. Composition selon l'une des revendications 7 à 23, **caractérisée en ce qu'**elle comprend au moins un adjuvant choisi parmi les tensioactifs anioniques, cationiques, non ioniques, amphotères, zwitterioniques ou leurs mélanges, les polymères anioniques, cationiques, non ioniques, amphotères, zwitterioniques ou leurs mélanges, les agents épaississants minéraux ou organiques, les agents antioxydants, les agents de pénétration, les agents séquestrants, les parfums, les tampons, les agents dispersants, les agents de conditionnement, les agents filmogènes, les céramides, les agents conservateurs, les agents opacifiants.

25. Composition selon l'une des revendications 7 à 24, **caractérisée en ce qu'**elle comprend au moins un agent oxydant choisi parmi le peroxyde d'hydrogène, le peroxyde d'urée, les bromates de métaux alcalins, les persels, les peracides et les enzymes oxydases.

26. Composition selon la revendication 25, **caractérisée en ce que** l'agent oxydant est le peroxyde d'hydrogène.

27. Procédé de teinture des fibres kératiniques, **caractérisé en ce qu'**il comprend l'application d'une composition selon l'une des revendications 7 à 26 sur les fibres kératiniques, puis une étape consistant à laisser poser pendant une période comprise entre 5 minutes et une heure, de préférence entre 15 minutes et 1 heure.

28. Procédé selon la revendication 27, **caractérisé en ce qu'**il comprend l'application d'une composition selon l'une des revendications 7 à 13 et 22 à 26 sur les fibres kératiniques, puis une étape consistant à laisser poser pendant une durée comprise entre 5 minutes et une heure, puis après un éventuel rinçage l'application d'une composition comprenant au moins un agent alcalin pendant un temps de pose compris entre quelques secondes et 30 minutes.

29. Procédé selon la revendication 27, **caractérisé en ce qu'**il comprend l'application d'une composition selon l'une des revendications 14 à 26, puis une étape consistant à laisser poser pendant une durée comprise entre 5 et 60 minutes, puis après un éventuel rinçage l'application d'une composition contenant au moins un agent oxydant et au moins un agent alcalin qu'on laisse poser de 5 minutes à 60 minutes.

30. Utilisation d'une composition selon l'une des revendications 7 à 26 pour la teinture des fibres kératiniques, en particulier des fibres kératiniques humaines telles que les cheveux.

31. Utilisation d'une composition selon l'une des revendications 7 à 26 sur les des fibres kératiniques, en particulier les fibres kératiniques humaines telles que les cheveux, pour obtenir des teintures présentant une bonne résistance aux agents extérieurs et aux shampooings.

## Claims

1. Use, as a direct dye in dye compositions for keratin fibres, in particular human keratin fibres such as hair, or for the manufacture of such compositions, of an organosilane-type compound of formula (I): 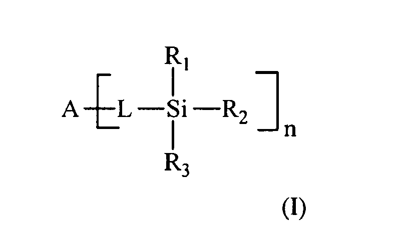 in which:
the group A is a group having a direct dyeing function,
the group L is a linking arm,
R₁, R₂, and R₃, independently of each other, represent a hydrogen atom; a mono- or polyhydroxyalkyl radical having from 1 to 10 carbon atoms; a mono- or polyaminoalkyl radical having from 1 to 10 carbon atoms; a mono- or polyhaloalkyl radical having from 1 to 10 carbon atoms; a mono- or polyalkoxyalkyl radical, the alkoxy radical being C₁-C₁₀ and the alkyl radical being C₁-C₁₀; a C₆-C₁₈ aryl radical; a C₆-C₁₈ mono- or polyaminoaryl radical; a C₆-C₁₈ mono- or polyhydroxyaryl radical; a mono- or polyalkoxyaryl radical, the alkoxy radical being C₁-C₁₀ and the aryl radical being C₆-C₁₈; an alkylaryl radical, the alkyl radical being C₁-C₁₀ and the aryl radical being C₆-C₁₈; an arylalkyl radical, the alkyl radical being C₁-C₁₀ and the aryl radical being C₆-C₁₈; a carboxyalkyl or sulphoalkyl radical, the alkyl radical being C₁-C₁₀,
n is an integer between 1 and 10.

2. Use according to Claim 1, **characterized in that** the organosilane-type compound is of formula (II): 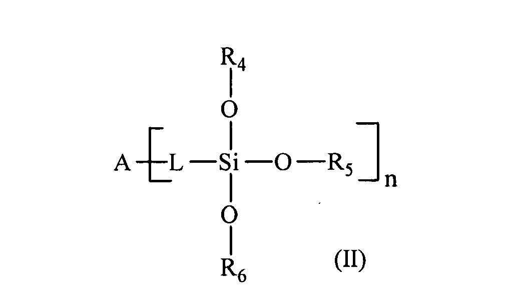 in which:
the group A is a group having a direct dyeing function,
the group L is a linking arm,
R₄, R₅ and R₆, independently of each other, represent a linear or branched, C₁-C₁₀, preferably C₁-C₆, alkyl radical,
n is an integer between 1 and 10.

3. Use according to Claim 1 or 2, **characterized in that** the group A is chosen from the radicals derived from aromatic nitro, anthraquinone, naphthoquinone, benzoquinone, azo, xanthene, triarylmethane, azine, thiazine, phenothiazine, diazine, phenodiazine, acridine, cyaninemethine, azomethine, nitro, phthalocyanine, indoaniline dyes, and natural direct dyes.

4. Use according to one of Claims 1 to 3, **characterized in that** the linking arm L is a linear or branched, C₁-C₂₀, preferably C₁-C₆, hydrocarbon chain, it being possible for one or more carbon atoms of the chain to be replaced by a heteroatom, it being possible for the chain to be saturated or unsaturated or to contain an arylene radical, a divalent terephthalamide radical, a divalent triazine radical, a radical -NHCO-.

5. Use according to Claim 4, **characterized in that** the hydrocarbon chain L is substituted with one or more hydroxyl, alkoxy, amino, alkylamino or halogen radicals.

6. Use according to either of Claims 4 and 5, **characterized in that** the linking arm L is a linear or branched C₁-C₂₀, preferably C₁-C₆, alkylene chain.

7. Dye composition for dyeing keratin fibres, in particular human keratin fibres such as hair, comprising in an appropriate dyeing medium at least one oxidation base and at least one organosilane-type compound of formula (I): 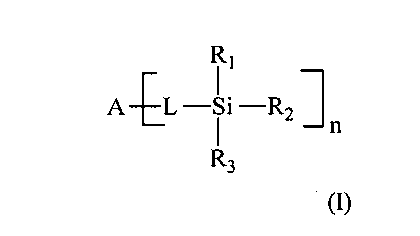 in which:
the group A is a group having a direct dyeing function,
the group L is a linking arm,
R₁, R₂, and R₃, independently of each other, represent a hydrogen atom; a mono- or polyhydroxyalkyl radical having from 1 to 10 carbon atoms; a mono- or polyaminoalkyl radical having from 1 to 10 carbon atoms; a mono- or polyhaloalkyl radical having from 1 to 10 carbon atoms; a mono- or polyalkoxyalkyl radical, the alkoxy radical being C₁-C₁₀ and the alkyl radical being C₁-C₁₀; a C₆-C₁₈ aryl radical; a C₆-C₁₈ mono- or polyaminoaryl radical; a C₆-C₁₈ mono- or polyhydroxyaryl radical; a mono- or polyalkoxyaryl radical, the alkoxy radical being C₁-C₁₀ and the aryl radical being C₆-C₁₈; an alkylaryl radical, the alkyl radical being C₁-C₁₀ and the aryl radical being C₆-C₁₈; an arylalkyl radical, the alkyl radical being C₁-C₁₀ and the aryl radical being C₆-C₁₈; a carboxyalkyl or sulphoalkyl radical, the alkyl radical being C₁-C₁₀,
n is an integer between 1 and 10.

8. Composition according to Claim 7, **characterized in that** the organosilane-type compound is of formula (II): 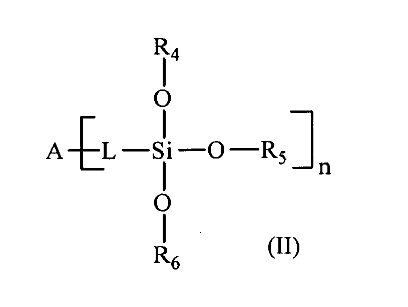 in which:
the group A is a group having a direct dyeing function,
the group L is a linking arm,
R₄, R₅ and R₆, independently of each other, represent a linear or branched, C₁-C₁₀, preferably C₁-C₆, alkyl radical,
n is an integer between 1 and 10.

9. Composition according to Claim 7 or 8, **characterized in that** the group A is chosen from the radicals derived from aromatic nitro, anthraquinone, naphthoquinone, benzoquinone, azo, xanthene, triarylmethane, azine, thiazine, phenothiazine, diazine, phenodiazine, acridine, cyaninemethine, azomethine, nitro, phthalocyanine, indoaniline dyes, and natural direct dyes.

10. Composition according to one of Claims 7 to 9, **characterized in that** the linking arm L is a linear or branched, C₁-C₂₀, preferably C₁-C₆, hydrocarbon chain, it being possible for one or more carbon atoms of the chain to be replaced by a heteroatom, it being possible for the chain to be saturated or unsaturated or to contain an arylene radical, a divalent terephthalamide radical, a divalent triazine radical, a radical -NHCO-.

11. Composition according to Claim 10, **characterized in that** the hydrocarbon chain L is substituted with one or more hydroxyl, alkoxy, amino, alkylamino or halogen radicals.

12. Composition according to either of Claims 10 and 11, **characterized in that** the linking arm is a linear or branched C₁-C₂₀, preferably C₁-C₆, alkylene chain.

13. Composition according to one of Claims 7 to 12, **characterized in that** it comprises from 0.001 to 20%, preferably from 0.01 to 10%, and more preferably still from 0.1 to 5% by weight of direct dye(s) of formula (I) relative to the total weight of the composition.

14. Composition according to one of Claims 7 to 13, **characterized in that** it comprises at least one oxidation dye precursor chosen from oxidation bases.

15. Composition according to Claim 14, **characterized in that** the oxidation base is chosen from para-phenylenediamines, bisphenylalkylenediamines, para-aminophenols, ortho-aminophenols, heterocyclic bases and their addition salts.

16. Dye composition according to either of Claims 14 and 15, **characterized in that** the oxidation base(s) are present in a quantity of between 0.001 to 20% by weight, and preferably between 0.005 and 6% by weight relative to the total weight of the composition.

17. Composition according to one of Claims 7 to 16, **characterized in that** it contains at least one oxidation dye precursor chosen from couplers.

18. Composition according to Claim 17, **characterized in that** the coupler is chosen from meta-phenylenediamines, meta-aminophenols, meta-diphenols, naphthalene couplers, heterocyclic couplers and their addition salts.

19. Composition according to Claim 18, **characterized in that** the coupler is chosen from 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)-propane, 3-ureidoaniline, 3-ureido-1-dimethylaminobenzene, sesamol, 1-β-hydroxyethylamino-3,4-methylenedioxybenzene, α-naphthol, 2-methyl-1-naphthol, 6-hydroxyindole, 4-hydroxyindole, 4-hydroxy-N-methylindole, 2-amino-3-hydroxypyridine, 6-hydroxybenzomorpholine, 3,5-diamino-2,6-dimethoxypyridine, 1-N-(β-hydroxyethyl)amino-3,4-methylenedioxybenzene, 2,6-bis(β-hydroxyethylamino)toluene and their addition salts.

20. Composition according to one of Claims 17 to 19, **characterized in that** the coupler(s) are present in a quantity of between 0.001 and 20%, preferably between 0.005 and 6% by weight relative to the total weight of the composition.

21. Composition according to one of Claims 7 to 20, **characterized in that** it comprises at least one additional direct dye.

22. Composition according to one of Claims 7 to 21, **characterized in that** it comprises at least one organic solvent.

23. Composition according to Claim 22, **characterized in that** the organic solvent is chosen from ethanol, propylene glycol, glycerol and polyol monoethers.

24. Composition according to one of Claims 7 to 23, **characterized in that** it comprises at least one adjuvant chosen from anionic, cationic, nonionic, amphoteric or zwitterionic surfactants or mixtures thereof, anionic, cationic, nonionic, amphoteric or zwitterionic polymers or mixtures thereof, inorganic or organic thickeners, antioxidants, penetrating agents, sequestrants, perfumes, buffers, dispersing agents, conditioning agents, film-forming agents, ceramides, preservatives, opacifying agents.

25. Composition according to one of Claims 7 to 24, **characterized in that** it comprises at least one oxidizing agent chosen from hydrogen peroxide, urea peroxide, alkali metal bromates, persalts, peracids and oxidase enzymes.

26. Composition according to Claim 25, **characterized in that** the oxidizing agent is hydrogen peroxide.

27. Method for dyeing keratin fibres, **characterized in that** it comprises the application of a composition according to one of Claims 7 to 26 to the keratin fibres, and then a step consisting in leaving in for a period of between 5 minutes and one hour, preferably between 15 minutes and 1 hour.

28. Method according to Claim 27, **characterized in that** it comprises the application of a composition according to one of Claims 7 to 13 and 22 to 26 to keratin fibres, and then a step consisting in leaving in for a period of between 5 minutes and one hour, and then after an optional rinse, the application of a composition comprising at least one alkaline agent for a leave-in time of between a few seconds and 30 minutes.

29. Method according to Claim 27, **characterized in that** it comprises the application of a composition according to one of Claims 14 to 26, and then a step consisting in leaving in for a period of between 5 and 60 minutes, and then after an optional rinse, the application of a composition containing at least one oxidizing agent and at least one alkaline agent which is left in for 5 minutes to 60 minutes.

30. Use of a composition according to one of Claims 7 to 26 for dyeing keratin fibres, in particular human keratin fibres such as hair.

31. Use of a composition according to one of Claims 7 to 26 on keratin fibres, in particular human keratin fibres such as hair, in order to obtain colours having good resistance to external agents and to shampoos.

## Patentansprüche

1. Verwendung einer Verbindung vom Organosilantyp der Formel (I) als Direktfarbstoff in Farbmittelzusammensetzungen für Keratinfasern und insbesondere menschliche Keratinfasern wie Haare oder für die Herstellung solcher Zusammensetzungen: 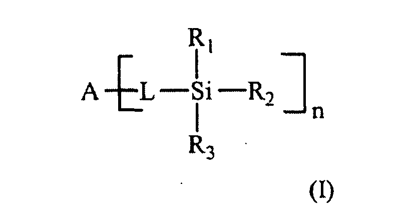 in der Formel:
· die Gruppe A ist eine Gruppe mit Direktfarbstofffunktion,
· die Gruppe L ist eine Verbindungsgruppe,
· die Gruppen R₁, R₂ und R₃ bedeuten unabhängig voneinander ein Wasserstoffatom; eine Mono- oder Polyhydroxyalkylgruppe mit 1 bis 10 Kohlenstoffatomen; eine Mono- oder Polyaminoalkylgruppe mit 1 bis 10 Kohlenstoffatomen; eine Mono- oder Polyhalogenalkylgruppe mit 1 bis 10 Kohlenstoffatomen; eine Mono- oder Polyalkoxyalkylgruppe, wobei die Alkoxygruppe 1 bis 10 Kohlenstoffatome aufweist und die Alkylgruppe 1 bis 10 Kohlenstoffatome aufweist; eine Arylgruppe mit 6 bis 18 Kohlenstoffatomen; eine Mono- oder Polyaminoarylgruppe mit 6 bis 18 Kohlenstoffatomen; eine Mono- oder Polyhydroxyarylgruppe mit 6 bis 18 Kohlenstoffatomen; eine Mono- oder Polyalkoxyarylgruppe, wobei die Alkoxygruppe 1 bis 10 Kohlenstoffatome aufweist und die Arylgruppe 6 bis 18 Kohlenstoffatome aufweist; eine Alkylarylgruppe, wobei die Alkylgruppe 1 bis 10 Kohlenstoffatome aufweist und die Arylgruppe 6 bis 18 Kohlenstoffatome aufweist; eine Arylalkylgruppe, wobei die Alkylgruppe 1 bis 10 Kohlenstoffatome aufweist und die Arylgruppe 6 bis 18 Kohlenstoffatome aufweist; eine Carboxyalkyl- oder Sulfoalkylgruppe, wobei die Alkylgruppe 1 bis 10 Kohlenstoffatome besitzt;
.n ist eine ganze Zahl im Bereich von 1 bis 10.

2. Verwendung nach Anspruch 1, **dadurch gekennzeichnet, dass** die Verbindung vom Organosilantyp der Formel (II) entspricht: 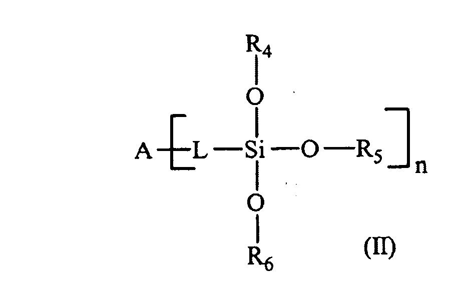 in der Formel:
· die Gruppe A ist eine Gruppe mit Direktfarbstofffunktion,
· die Gruppe L ist eine Verbindungsgruppe,
· die Gruppen R₄, R₅ und R₆ bedeuten unabhängig voneinander eine geradkettige oder verzweigte Alkylgruppe mit 1 bis 10 Kohlenstoffatomen und insbesondere 1 bis 6 Kohlenstoffatomen;
· n ist eine ganze Zahl im Bereich von 1 bis 10.

3. Verwendung nach Anspruch 1 oder 2, **dadurch gekennzeichnet, dass** die Gruppe A unter den Gruppen ausgewählt ist, die von aromatischen Nitrofarbstoffen, Anthrachinon-Farbstoffen, Naphthochinon-Farbstoffen, Benzochinon-Farbstoffen, Azofarbstoffen, Xanthen-Farbstoffen, Triarylmethan-Farbstoffen, A-zin-Farbstoffen, Thiazin-Farbstoffen, Phenothiazin-Farbstoffen, Diazin-Farbstoffen, Phenodiazin-Farbstoffen, Acridin-Farbstoffen, Cyaninmethin-Farbstoffen, Azomethin-Farbstoffen, nitrierten Farbstoffen, Phthalocyanin-Farbstoffen, Indoanilin-Farbstoffen und den natürlichen direktziehenden Farbstoffen ausgewählt ist.

4. Verwendung nach einem der Ansprüche 1 bis 3, **dadurch gekennzeichnet, dass** die Verbindungsgruppe L eine geradkettige oder verzweigte Kohlenwasserstoffkette mit 1 bis 20 Kohlenstoffatomen und vorzugsweise 1 bis 6 Kohlenstoffatomen bedeutet, wobei ein oder mehrere Kohlenstoffatome der Kette durch ein Heteroatom ersetzt sein können, die Kette gesättigt oder ungesättigt sein kann, oder eine Arylengruppe, eine zweiwertige Terephthalamidgruppe, eine zweiwertige Triazingruppe oder eine Gruppe NHCO enthalten kann.

5. Verwendung nach Anspruch 4, **dadurch gekennzeichnet, dass** die Kohlenwasserstoffkette L mit einer oder mehreren Gruppen Hydroxy, Alkoxy, Amino, Alkylamino oder Halogen substituiert ist.

6. Verwendung nach einem der Ansprüche 4 oder 5, **dadurch gekennzeichnet, dass** die Verbindungsgruppe L eine geradkettige oder verzweigte Alkylengruppe mit 1 bis 20 Kohlenstoffatomen und vorzugsweise 1 bis 6 Kohlenstoffatomen ist.

7. Farbmittelzusammensetzung zum Färben von Keratinfasern und insbesondere menschlichen Keratinfasern wie Haaren, die in einem zum Färben geeigneten Medium mindestens eine Oxidationsbase und mindestens eine Verbindung vom Organosilantyp der Formel (I) enthält: 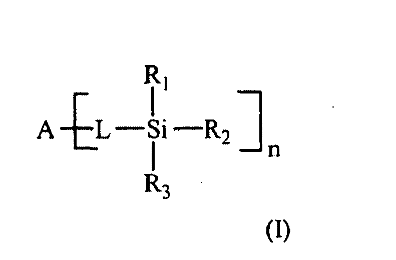 in der Formel:
· die Gruppe A ist eine Gruppe mit Direktfarbstofffunktion,
· die Gruppe L ist eine Verbindungsgruppe,
· die Gruppen R₁, R₂ und R₃ bedeuten unabhängig voneinander ein Wasserstoffatom; eine Mono- oder Polyhydroxyalkylgruppe mit 1 bis 10 Kohlenstoffatomen; eine Mono- oder Polyaminoalkylgruppe mit 1 bis 10 Kohlenstoffatomen; eine Mono- oder Polyhalogenalkylgruppe mit 1 bis 10 Kohlenstoffatomen; eine Mono- oder Polyalkoxyalkylgruppe, wobei die Alkoxygruppe 1 bis 10 Kohlenstoffatome aufweist und die Alkylgruppe 1 bis 10 Kohlenstoffatome aufweist; eine Arylgruppe mit 6 bis 18 Kohlenstoffatomen; eine Mono- oder Polyaminoarylgruppe mit 6 bis 18 Kohlenstoffatomen; eine Mono- oder Polyhydroxyarylgruppe mit 6 bis 18 Kohlenstoffatomen; eine Mono- oder Polyalkoxyarylgruppe, wobei die Alkoxygruppe 1 bis 10 Kohlenstoffatome aufweist und die Arylgruppe 6 bis 18 Kohlenstoffatome aufweist; eine Alkylarylgruppe, wobei die Alkylgruppe 1 bis 10 Kohlenstoffatome aufweist und die Arylgruppe 6 bis 18 Kohlenstoffatome aufweist; eine Arylalkylgruppe, wobei die Alkylgruppe 1 bis 10 Kohlenstoffatome aufweist und die Arylgruppe 6 bis 18 Kohlenstoffatome aufweist; eine Carboxyalkyl- oder Sulfoalkylgruppe, wobei die Alkylgruppe 1 bis 10 Kohlenstoffatome besitzt;
· n ist eine ganze Zahl im Bereich von 1 bis 10.

8. Zusammensetzung nach Anspruch 7, **dadurch gekennzeichnet, dass** die Verbindung vom Organosilantyp der Formel (II) entspricht: 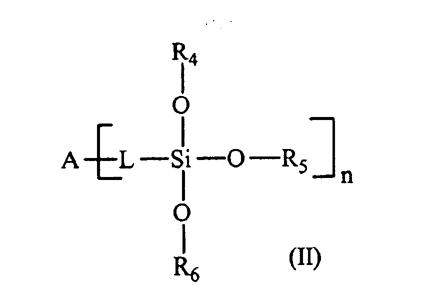 in der Formel:
· die Gruppe A ist eine Gruppe mit Direktfarbstofffunktion,
· die Gruppe L ist eine Verbindungsgruppe,
· die Gruppen R₄, R₅ und R₆ bedeuten unabhängig voneinander eine geradkettige oder verzweigte Alkylgruppe mit 1 bis 10 Kohlenstoffatomen und insbesondere 1 bis 6 Kohlenstoffatomen;
· n ist eine ganze Zahl im Bereich von 1 bis 10.

9. Zusammensetzung nach Anspruch 7 oder 8, **dadurch gekennzeichnet, dass** die Gruppe A unter den Gruppen ausgewählt ist, die von den aromatischen Nitrofarbstoffen, Anthrachinon-Farbstoffen, Naphthochinon-Farbstoffen, Benzochinon-Farbstoffen, Azofarbstoffen, Xanthen-Farbstoffen, Triarylmethan-Farbstoffen, Azin-Farbstoffen, Thiazin-Farbstoffen, Phenothiazin-Farbstoffen, Diazin-Farbstoffen, Phenodiazin-Farbstoffen, Acridin-Farbstoffen, Cyaninmethin-Farbstoffen, Azomethin-Farbstoffen, nitrierten Farbstoffen, Phthalocyanin-Farbstoffen, Indoanilin-Farbstoffen und den natürlichen direktziehenden Farbstoffen ausgewählt ist.

10. Zusammensetzung nach einem der Ansprüche 7 bis 9, **dadurch gekennzeichnet, dass** die Verbindungsgruppe L eine geradkettige oder verzweigte Kohlenwasserstoffkette mit 1 bis 20 Kohlenstoffatomen und vorzugsweise 1 bis 6 Kohlenstoffatomen bedeutet, wobei ein oder mehrere Kohlenstoffatome der Kette durch ein Heteroatom ersetzt sein können, die Kette gesättigt oder ungesättigt sein kann, oder eine Arylengruppe, eine zweiwertige Terephthalamidgruppe, eine zweiwertige Triazingruppe oder eine Gruppe NHCO enthalten kann.

11. Zusammensetzung nach Anspruch 10, **dadurch gekennzeichnet, dass** die Kohlenwasserstoffkette L mit einer oder mehreren Gruppen Hydroxy, Alkoxy, Amino, Alkylamino oder Halogen substituiert ist.

12. Zusammensetzung nach einem der Ansprüche 10 und 11, **dadurch gekennzeichnet, dass** die Verbindungsgruppe L eine geradkettige oder verzweigte Alkylengruppe mit 1 bis 20 Kohlenstoffatomen und vorzugsweise 1 bis 6 Kohlenstoffatomen ist.

13. Zusammensetzung nach einem der Ansprüche 7 bis 12, **dadurch gekennzeichnet, dass** sie 0,001 bis 20 Gew.-%, vorzugsweise 0,01 bis 10 Gew.-% und besonders bevorzugt 0,1 bis 5 Gew.-% Direktfarbstoff(e) der Formel (I), bezogen auf das Gesamtgewicht der Zusammensetzung, enthält.

14. Zusammensetzung nach einem der Ansprüche 7 bis 13, **dadurch gekennzeichnet, dass** sie mindestens eine Farbstoffvorprodukt eines Oxidationsfarbstoffes enthält, das unter den Oxidationsbasen ausgewählt ist.

15. Zusammensetzung nach Anspruch 14, **dadurch gekennzeichnet, dass** die Oxidationsbase unter den *p*-Phenylendiaminen, Bisphenylalkylendiaminen, *p*-Aminophenolen, o-Aminophenolen und den heterocyclischen Basen und deren Additionssalzen ausgewählt ist.

16. Zusammensetzung nach einem der Ansprüche 14 und 15, **dadurch gekennzeichnet, dass** die Oxidationsbase oder die Oxidationsbasen in einem Mengenanteil von 0,001 bis 20 Gew.-% und vorzugsweise 0,005 bis 6 Gew.-%, bezogen auf das Gesamtgewicht der Zusammensetzung, enthalten sind.

17. Zusammensetzung nach einem der Ansprüche 7 bis 16, **dadurch gekennzeichnet, dass** sie mindestens eine Farbstoffvorprodukt eines Oxidationsfarbstoffes enthält, das unter den Kupplern ausgewählt ist.

18. Zusammensetzung nach Anspruch 17, **dadurch gekennzeichnet, dass** der Kuppler unter den *m*-Phenylendiaminen, *m*-Aminophenolen, *m*-Dihydroxybenzolen, Naphthalinkupplern und heterocyclischen Kupplern und deren Additionssalzen ausgewählt ist.

19. Zusammensetzung nach Anspruch 18, **dadurch gekennzeichnet, dass** der Kuppler unter 1,3-Dihydroxybenzol, 1,3-Dihydroxy-2-methylbenzol, 4-Chlor-1,3-dihydroxybenzol, 2,4-Diamino-1-(β-hydroxyethyloxy)-benzol, 2-Amino-4-(β-hydroxyethylamino)-1-methoxybenzol, 1,3-Diaminobenzol, 1,3-Bis(2,4-diaminophenoxy)propan, 3-Ureidoanilin, 3-Ureido-1-dimethylaminobenzol, Sesamol, 1-β-Hydroxyethylamino-3,4-methylendioxybenzol, α-Naphthol, 2-Methyl-1-naphthol, 6-Hydroxyindol, 4-Hydroxyindol, 4-Hydroxy-N-methylindol, 2-Amino-3-hydroxy-pyridin, 6-Hydroxybenzomorpholin, 3,5-Diamino-2,6-dimethoxypyridin, 1-N-(β-Hydroxyethyl)amino-3,4-methylendioxybenzol, 2,6-Bis(β-hydroxyethylamino)toluol und deren Additionssalzen ausgewählt ist.

20. Zusammensetzung nach einem der Ansprüche 17 bis 19, **dadurch gekennzeichnet, dass** der oder die Kuppler in einem Mengenanteil von 0,001 bis 20 Gew.-% und vorzugsweise 0,005 bis 6 Gew.-%, bezogen auf das Gesamtgewicht der Zusammensetzung, enthalten sind.

21. Zusammensetzung nach einem der Ansprüche 7 bis 20, **dadurch gekennzeichnet, dass** sie ferner mindestens einen ergänzenden Direktfarbstoff enthält.

22. Zusammensetzung nach einem der Ansprüche 7 bis 21, **dadurch gekennzeichnet, dass** sie ferner mindestens ein organisches Lösemittel enthält.

23. Zusammensetzung nach Anspruch 22, **dadurch gekennzeichnet, dass** das organische Lösemittel unter Ethanol, Propylenglycol, Glycerin und Polyolmonoethern ausgewählt ist.

24. Zusammensetzung nach einem der Ansprüche 7 bis 23, **dadurch gekennzeichnet, dass** sie mindestens einen Zusatzstoff enthält, der unter den anionischen, kationischen, nichtionischen, amphoteren, zwitterionischen grenzflächenaktiven Stoffen oder deren Gemischen, anionischen, kationischen, nichtionischen, amphoteren, zwitterionischen Polymeren oder deren Gemischen, anorganischen oder organischen Verdickungsmitteln, Antioxidantien, Penetrationsmitteln, Maskierungsmitteln, Parfums, Puffern, Dispergiermitteln, Konditioniermitteln, Filmbildnern, Ceramiden, Konservierungsmitteln und Trübungsmitteln ausgewählt ist.

25. Zusammensetzung nach einem der Ansprüche 7 bis 24, **dadurch gekennzeichnet, dass** sie mindestens ein Oxidationsmittel enthält, das unter Wasserstoffperoxid, Harnstoffperoxid, Alkalimetallbromaten, Salzen von Persäuren, Persäuren und Oxidasen ausgewählt ist.

26. Zusammensetzung nach Anspruch 25, **dadurch gekennzeichnet, dass** es sich bei dem Oxidationsmittel um Wasserstoffperoxid handelt.

27. Verfahren zum Färben von Keratinfasern, **dadurch gekennzeichnet, dass** es umfasst, eine Zusammensetzung nach einem der Ansprüche 7 bis 26 auf die Keratinfasern aufzutragen und anschließend ferner einen Schritt aufweist, der darin besteht, während einer Zeitspanne von 5 Minuten bis 1 Stunde und vorzugsweise 15 Minuten bis 1 Stunde einwirken zu lassen.

28. Verfahren nach Anspruch 27, **dadurch gekennzeichnet, dass** es umfasst, eine Zusammensetzung nach einem der Ansprüche 7 bis 13 und 22 bis 26 auf die Keratinfasern aufzutragen und anschließend ferner einen Schritt aufweist, der darin besteht, während einer Zeitspanne von 5 Minuten bis 1 Stunde einwirken zu lassen, worauf, nachdem gegebenenfalls gespült wurde, während einer Zeitspanne im Bereich von einigen Sekunden bis 30 Minuten eine Zusammensetzung aufgetragen wird, die mindestens einen alkalischen Stoff enthält.

29. Verfahren nach Anspruch 27, **dadurch gekennzeichnet, dass** es umfasst, eine Zusammensetzung nach einem der Ansprüche 14 bis 26 auf die Keratinfasern aufzutragen und anschließend ferner einen Schritt aufweist, der darin besteht, während einer Zeitspanne von 5 bis 60 Minuten einwirken zu lassen, worauf, nachdem gegebenenfalls gespült wurde, während einer Zeitspanne von 5 bis 60 Minuten eine Zusammensetzung aufgetragen wird, die mindestens ein Oxidationsmittel und mindestens einen alkalischen Stoff enthält.

30. Verwendung einer Zusammensetzung nach einem der Ansprüche 7 bis 26 zum Färben von Keratinfasern und insbesondere menschlichen Keratinfasern wie Haaren.

31. Verwendung einer Zusammensetzung nach einem der Ansprüche 7 bis 26 an Keratinfasern und insbesondere menschlichen Keratinfasern wie Haaren, um Färbungen zu bilden, die eine hohe Beständigkeit gegenüber von außen einwirkenden Agentien und Haarwäschen aufweisen.
